# EUROPÄISCHE PATENTANMELDUNG

(11) **EP 0 668 578 A2**
(43) Veröffentlichungstag der Anmeldung: **23.08.1995**
(21) Anmeldenummer: 95101473.7
(22) Anmeldetag: 03.02.1995
(51) Int. Cl.: G07F 7/10, G06K 19/10

(54) **Speicher- und selektives Informationsübermittlungssystem für persönliche Daten**

(30) Priorität: 16.02.1994 DE 4404841
(71) Anmelder: BAYER AG, 51368 Leverkusen (DE)
(72) Erfinder: Ahrens, Wolfgang, Dr., D-51515 Kürten (DE); Hartmann, Georg, Dr., D-51061 Köln (DE); Weikert, Gunnar, Dr., D-40589 Düsseldorf (DE)

(57) **Zusammenfassung**

Speicher- und selektives Informationsübermittlungssystem persönlicher Daten bestehend aus mindestens einer optischen Speicherkarte, auf der ein definiertes Speicherfeld für die Speicherung einer Vielzahl von Schlüsselbegriffen vorgesehen ist, einer Vielzahl von mindestens je einem Schlüsselbegriff zugeordneter Codes, mindestens einem Schreib/Lese-Gerät für die optische Speicherkarte mit einer Vielzahl von Schlüssel-Erkennungsfunktionen, wobei je eine Schlüsselerkennungsfunktion auf je eine von einer Vielzahl von in dem Schreib/Lese-Gerät enthaltenden Formatierungsfunktionen verweist, und diese im Zusammenwirken mit dem jeweils zugeordneten Code aktiviert, wobei jede Formatierungsfunktion auf ein Datenspeicherfeld der optischen Karte verweist und aufgrund der dazu gehörenden Formatangaben zum Lesen der dort gespeicherten Daten qualifiziert ist.

## Beschreibung

Die vorliegende Erfindung betrifft ein Speicher-und selektives Informationsübermittlungssystem für persönliche Daten auf Basis einer optischen Speicherkarte.

Optische Speicherkarten im Kreditkartenformat, die mittels Laserstrahlen beschrieben ("WORM" = write once read many times memory) und gelesen werden können, haben aufgrund technischer Entwicklungen in den vergangenen Jahren Speicherkapazitäten von mehreren Megabyte, insbesondere 4 bis 6 Megabyte erzielt. Eine entsprechende Entwicklung haben die Schreib/Lese-Geräte genommen. Im Gegensatz zur magnetischen Speicherung auf Disketten sind die optisch gespeicherten Daten unempfindlich gegen mechanische und elektromagnetische Einflüsse. Sie sind also geeignet, z.B. in Geldbörsen, an der Person mitgeführt zu werden. Das zum Beschreiben und Lesen der Karte erforderliche Schreib/Lese-Gerät kann in Form eines handlichen Zusatzgerätes zu in vielen Haushalten bereits vorhandenen Personalcomputern ausgebildet werden. Die Technologie der persönlichen Karte, die es erlaubt jegliche Informationen ständig mit sich zu führen, ist demgemäß verfügbar (z.B. US-A 49 37 963).

Ein Vorteil der optischen bwz. magnetooptischen Karte besteht ferner darin, daß im Falle von Verlust oder wenn die Karte sonstwie in fremde Hände gerät, die gespeicherten Daten nicht ohne weiteres lesbar sind. Vielmehr ist hierzu das Schreib/Lese-Gerät erforderlich.

So wurde bereits vorgeschlagen (z.B. EP-A-467 693), die optische Karte zur Speicherung medizinischer Informationen zu nutzen, so daß der Arzt im Falle einer medizinischen Behandlung über das bei ihm installierte Schreib/Lese-Gerät Einsicht in die Anamnese erhält. Ferner wurde bereits vorgeschlagen, die optische Karte zur Speicherung von Röntgenbildnern einzusetzen, so daß die Röntgenhäufigkeit und damit die Dosisbelastung des Patienten reduziert wird und ferner gegebenenfalls Röntgenbilder über einen längeren Zeitraum ständig verfügbar bleiben (DE-C 41 33 718, DE-A 42 03 447).

Wünschenswert wäre es, nicht nur medizinische Daten sondern alle denkbaren persönlichen Daten auf der Speicherkarte mit sich zu führen. So ist ein häufig unterschätztes Problem die Aufbewahrung persönlicher Akten, Versicherungspolicen, Zeugnisse und ähnliches gegen Naturkatastrophen (Überschwemmung, Feuer, auch Diebstahl) ungeschützt in der häuslichen Wohnung, da solche Unterlagen häufig nur sehr schwer wiederzubeschaffen sind und je nach Art des Verlustes auch die Abwicklung von Versicherungsschäden bei Unkenntnis des Versicherers und der Versicherungsnummer erschwert wird.

Die optische Karte ist zur Speicherung all solcher Daten geeignet. Ihre allgemeine Einführung und Akzeptanz scheitert jedoch weitgehend an einer noch fehlenden Datensicherheit, indem nämlich befürchtet werden muß, daß bei jedem Lesevorgang, z.B. in der Arztpraxis, auch persönliche Informationen zugänglich gemacht werden, die im Interesse des Karteninhabers geheimzuhalten wären. Selbst wenn nur medizinische Daten gespeichert wurden, liegt es gelegentlich nicht im Interesse des Patienten, z.B. dem Zahnarzt Daten des Internisten zugänglich zu machen. Die prinzipielle Möglichkeit, eine Vielzahl von optischen Karten für unterschiedliche Informationsspeicherungen mit sich zu führen, wurde einerseits der Ausnutzung der hohen Speicherkapazität der Karte entgegenwirken und andererseits der Möglichkeit entgegenwirken, alle Informationen regelmäßig mit sich zu führen.

Es besteht also ein Bedarf, ein selektives Informationsübermittlungssystem auf Basis nur einer persönlichen optischen Speicherkarte zur Verfügung zu stellen, das es erlaubt, den Zugriff zu jeweils nur bestimmten, auf der Karte gespeicherten Daten zu autorisieren und die übrigen von einer Leseoperation auszuschließen.

Gegenstand der vorliegenden Erfindung ist demgemäß ein Speicher- und selektives Informationsübermittlungssystem persönlicher Daten, bestehend aus einer optischen Speicherkarte, auf der ein räumlich definiertes Speicherfeld für die ausschließliche Speicherung einer Vielzahl von Schlüsselbegriffen vorgesehen ist, ferner je mindestens ein weiterer, jeweils für mindestens je einen Schlüsselbegriff berechtigender Code vorgesehen ist, sowie mindestens ein Schreib/Lese-Gerät für die Speicherkarte mit einer Vielzahl von Schlüssel- Erkennungsfunktionen, wobei je eine Schlüsselerkennungsfunktion auf je eine von einer Vielzahl von Formatierungsfunktionen (im kryptographischen Sinne: Dechiffrierfunktion, die aus dem Code einen Dechiffrierparameter ermittelt, mit dessen Hilfe das Datenspeicherfeld dechiffrierbar, d.h. lesbar, wird) verweist und diese im Zusammenwirken mit dem jeweiligen Code aktiviert, wobei jede Formatierungsfunktion auf ein Datenspeicherfeld der optischen Karte verweist und das Schreib/Lese-Gerät zum Lesen der dort gespeicherten Daten qualifiziert. Der nun gemeinsam mit dem auf der Karte gespeicherte Schlüsselbegriff zum Lesen qualifizierende Code kann z.B. so ausgebildet sein, daß schlüsselbegriffspezifisch über eine Tastatur ein Code-Wort bzw. eine Code-Ziffer (Dechiffrierparameter) dem Schreib/Lese-Gerät mitgeteilt wird.

In einer weiteren, bevorzugten Ausführungsform der vorliegenden Erfindung, ist eine von der optischen Speicherkarte getrennte Code-Karte vorgesehen, die eine Vielzahl von je mindestens einem Schlüsselbegriff zugeordneten Codebegriffen speichert und z.B. über einen Codekarten-Leser mit Leseschlitz dem Schreib/Lese-Gerät mitgeteilt werden können. Dabei können die unterschiedlichen Codebegriffe in Form von Magnetstreifen oder optisch lesbaren Balkencodes auf der Code- karte gespeichert sein. Zum Beispiel kann die Codekarte beidseitig jeweils parallel zu den Kanten Balkencodes enthalten, wobei je ein Balkencode im Zusammenwirken mit mindestens je einem Schlüsselbegriff das Lesegerät aktiviert.

Der Inhaber der optischen Speicherkarte kann diese nun zum Zwecke der Zurverfügungstellung bestimmter Informationen aus der Hand geben, und dabei selbst, indem er die Codekarte nicht aus der Hand gibt, sondern sie selbst mit der gewünschten Seite und Kante durch den Leseschlitz schiebt, nur dasjenige optische Speicherfeld zugänglich machen, dessen Informationsinhalt er übermitteln will. Eine weiter bevorzugte Ausführungsform der vorliegenden Erfindung sieht vor, daß die Vielzahl der Codes die im Zusammenwirken mit den, Schlüsselbegriff der optischen Speicherkarte das Lesegerät aktivieren, jeweils zweiteilig ausgeführt sind, wobei die zweiteiligen Codes aus einem einheitlichen Passwort und einem auf der Codekarte gespeicherten jeweils unterschiedlichen Codebegriff bestehen.

Auf diese Weise ist sichergestellt, daß auch bei gleichzeitigem Verlust von Speicherkarte und Codekarte mangels Kenntnis des Passwortes kein Zugang zu den gespeicherten Informationen möglich wird.

Das Zusammenwirken von Schlüsselbegriff und Code kann dabei so gestaltet sein, daß über ein Hilfsprogramm im Schreib/Lese-Gerät der gespeicherte Schlüsselbegriff unter Verarbeitung mit dem Code so umgesetzt wird, daß dieser erst nach der Umsetzung mit dem Code auf eine Formatierungsfunktion verweist.

In einer weiter bevorzugten Ausführungsform der vorliegenden Erfindung ist die im Schreib/Lese-Gerät vorhandene Formatierungsfunktion unvollständig und wird erst nach dem Lesen des Codes durch ein im Schreib/Lese-Gerät vorhandenes Hilfsprogramm in die zu dem jeweiligen Datenspeicherfeld der optischen Karte gehörenden Formatangaben (im Sinne von Dechiffrierparameter) umgesetzt.

Auf diese Weise wird sichergestellt, daß auch durch Manipulation an dem herstellerseitig auf das Zusammenwirken von Schlüssel-Erkennungsfunktionen und Codes eingerichteten Schreib/Lese-Gerät dieses nicht für das mißbräuchliche Lesen von Datenspeicherfeldern eingerichtet werden kann.

Herstellerseitig kann vorgesehen sein, bestimmte Schlüsselerkennungs- und Formatierungsfunktionen nur den Schreib/Lese-Geräten bestimmter Personen zur Verfügung zu stellen. So kann ein Datenfeld für medizinische Daten einer bestimmten Indikation vorgesehen sein, und ein anderes Datenfeld für die Speicherung von Angaben über Daten und Aufbewahrungsort eines Testamentes. Die zugehörigen Schlüsselerkennungsfunktionen und Formatierungsfunktionen können dann nur den für diese Indikation zuständigen Ärzten bzw. den für Testamentsfragen zuständigen Notaren für deren Schreib/Lese-Geräte zur Verfügung gestellt werden. Das Schreib/Lese-Gerät kann über Passwort und/oder persönliche Berechtigungskarte gegen Benutzung durch Dritte in üblicher Weise gesichert sein.

Darüber hinaus können Schlüsselbegriffe und Datenfelder auf der optischen Speicherkarte vorgesehen sein, die auch ohne den Code, d.h. ohne Mitwirkung des Karteninhabers, von hierzu speziell berechtigten Lesegeräten gelesen werden können. Solche Daten können z.B. medizinische Daten sein, die im Falle eines Unfalls bei der Erstversorgung zu berücksichtigen sind. Mit entsprechenden Schlüsselerkennungsfunktionen und Formatierungsfunktionen bzw. Formatangaben würden dann die (und nur die) Schreib/Lese-Geräte der Unfallstationen und Notärzte ausgerüstet.

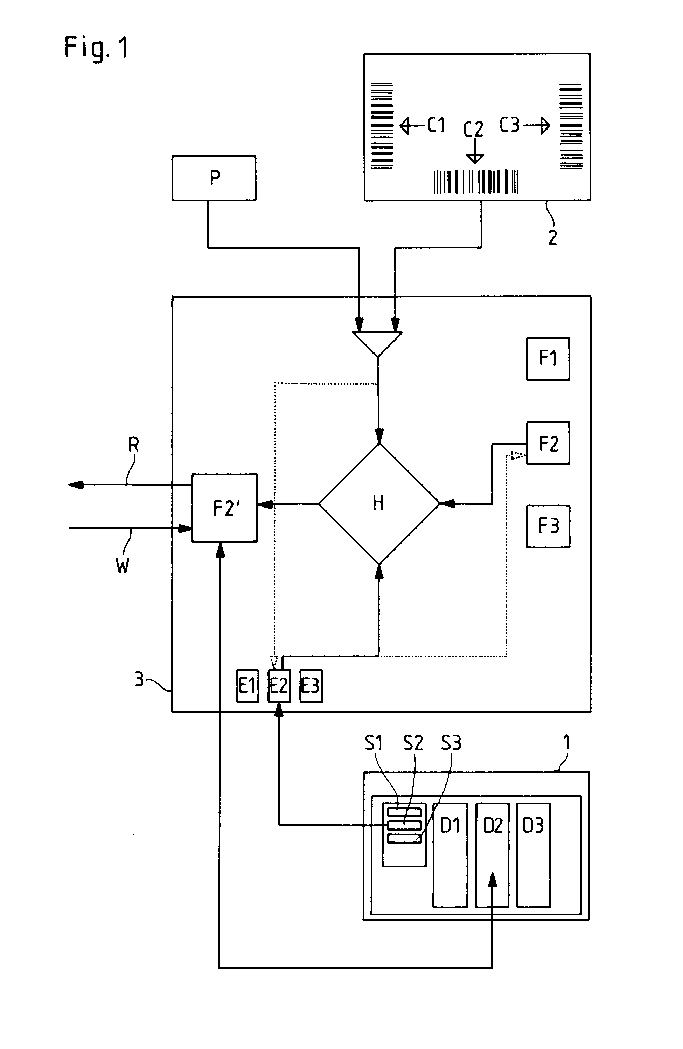

Die Erfindung wird in ihrer besonders bevorzugten Ausführungsform anhand der Fig. 1 näher erläutert. Die optische Speicherkarte 1 enthält ein Speicherfeld für Schlüsselbegriffe S1, S2 und S3, ferner den jeweiligen Schlüsselbegriffen zugeordnete Datenfelder D1, D2 und D3. Die Codekarte 2 enthält an ihrem Rand Balkencodes C1, C2 und C3. Das Schreib/Lese-Gerät 3 enthält Formatierungsfunktionen (Dechiffrierfunktionen) F1, F2 und F3. Passwort P und Code C2 werden in das Schreib/Lese-Gerät 3 eingelesen, das ferner über die Schlüsselerkennungsfunktion E2 den Schlüssel S2 liest. Durch Verarbeitung von P, C2 und S2 mit dem Hilfsprogramm H wird die Formatierungsfunktion F2 (Dechiffrierfunktion) zu den Formatangaben (Dechiffrierparametern) F2' umgesetzt, mit deren Hilfe das Speicherfeld D2 der optischen Speicherkarte 1 (Pfeil R) gelesen werden kann. Natürlich wird das Feld D2 ebenfalls mit den Formatangaben F2' (Chiffrierparametern) beschrieben (Pfeil W), so daß es, wie angegeben, wieder lesbar ist.

## Patentansprüche

1. Speicher- und selektives Informationsübermittlungssystem persönlicher Daten bestehend aus mindestens einer optischen Speicherkarte, auf der ein definiertes Speicherfeld für die Speicherung einer Vielzahl von Schlüsselbegriffen vorgesehen ist, einer Vielzahl -von mindestens je einem Schlüsselbegriff zugeordneter Codes, mindestens einem Schreib/Lese-Gerät für die optische Speicherkarte mit einer Vielzahl von Schlüssel-Erkennungsfunktionen, wobei je eine Schlüsselerkennungsfunktion auf je eine von einer Vielzahl von in dem Schreib/Lese-Gerät enthaltenden Formatierungsfunktionen verweist, und diese im Zusammenwirken mit dem jeweils zugeordneten Code aktiviert, wobei jede Formatierungsfunktion auf ein Datenspeicherfeld der optischen Karte verweist und aufgrund der dazu gehörenden Formatangaben zum Lesen der dort gespeicherten Daten qualifiziert ist.

2. Informationsübermittlungssystem nach Anspruch 1, wobei die Vielzahl der Codes auf einer der optischen Speicherkarte zugeordneten Codekarte gespeichert sind.
